Europäisches Patentamt

(19) European Patent Office

Office européen des brevets

(11) Publication number: **0 013 606**
**B1**

(12) **EUROPEAN PATENT SPECIFICATION**

(45) Date of publication of patent specification: 02.05.84

(51) Int. Cl.³: **A 61 K 9/22, A 61 K 9/26, A 61 K 9/70**

(21) Application number: **80300038.9**

(22) Date of filing: **04.01.80**

(54) Drug delivery device and method for its preparation.

(30) Priority: **11.01.79 US 2565**
**11.06.79 US 47084**
**14.08.79 JP 103495/79**

(43) Date of publication of application:
**23.07.80 Bulletin 80/15**

(45) Publication of the grant of the patent:
**02.05.84 Bulletin 84/18**

(84) Designated Contracting States:
**AT BE CH DE FR GB IT LU NL SE**

(56) References cited:
**FR - A - 2 437 830**
**GB - A - 1 108 837**
**GB - A - 2 021 950**
**US - A - 3 598 123**
**US - A - 4 076 798**

(73) Proprietor: **Key Pharmaceuticals, Inc.**
**50 N.W. 176th Street**
**Miami, Florida 33169 (US)**

(72) Inventor: **Keith, Alec D.**
**Pennsylvania State University Biophysics**
**618 Life-Sc. Lab. Univ. Park PA 16802 (US)**
Inventor: **Snipes, Wallace**
**Pennsylvania State University Biophysics**
**618 Life-Sc. Lab. Univ. Park PA 16802 (US)**

(74) Representative: **Werner, Hans-Karsten, Dr. et al,**
**Deichmannhaus am Hauptbahnhof**
**D-5000 Köln 1 (DE)**

Courier Press, Leamington Spa, England.

# 0 013 606

## Drug delivery device and method for its preparation

This invention relates to a drug delivery system for sustained release of a drug to a patient.

From GB—A—1,108,837 a local anesthetic material is known for topical application comprising a sheet-like structure of a film-forming material. Uniformly distributed throughout the structure is a local anesthetic agent. The film-forming material, however, is water-soluble and, only on dissolution, releases the local anesthetic agent for the action at the site of application. The material according to this prior art does not work under conditions under which the film does not dissolve into a moist surface.

From US—A—4,076,798 a pharmaceutical composition is known for the controlled continuous administration of the pre-determined dosage of a drug to a living animal, comprising a normally solid but biodegradable, hydrolyzable polyester resin of diglycolic acid and an unhindered glycol, which provides self-supporting film-forming properties. Also this product does not release the active ingredient unless the polyester resin is biodegraded and hydrolized. The product biodegrades at the surface only, thereby gradually eroding and simultaneously releasing the drug so that the area of release remains substantially constant during degradation.

GB—A—2,021,950 corresponding to DE—A—29 22 792 is not pre-published. In these documents there is described a medical bandage for use in continuous administration of nitroglycerin through the external body skin, wherein the nitroglycerin is together with a carrier medium which is permeable to the passage of nitroglycerin. The carrier medium herein is a lanolin-petrolatum formulation or a non-greasy ointment formulation together with a polymeric powder, preferably from polyethylene or other polyalkylenes. This material is different from the polymeric diffusion matrix according to this invention which releases active ingredient, as for example nitroglycerin, by the substantially constant diffusion to the dry skin.

Also FR—A—2,437,830 is not pre-published and describes a microporous polymer as cellulose triacetate, wherein trinitroglycerin is deposited. Also this product is different from the drug delivery system according to this invention.

From US—A—3,598,123 there is known a medical bandage for use in the administration of systemically active drugs by absorption through the external body skin or mucosa, wherein the systemically active drug is in a plurality of discrete microcapsules. Also these products are quite different from the polymeric diffusion matrix according to this invention.

According to the present invention a drug delivery system for sustained release of a drug is provided wherein the said system comprises:

(A) a polymeric diffusion matrix comprising from 2 to 60% of the glycerol, from 2 to 15% of polyvinylalcohol, from 2 to 10% of a water-soluble polymer with hydration sites, and the balance water, the percentages being by weight, and
(B) a therapeutically effective amount of at least one drug suitable for transdermal delivery to a patient, said drug component (B) being dispersed in said matrix (A) or being deposited in a drug reservoir attached to said matrix (A).

Agar, agarose, water-soluble cellulose derivatives or other compatible substances may replace all or part of the polyvinylpyrrolidone.

The polymeric drug diffusion matrix may be used in the uncured or in the cured state, but the uncured state is preferred. The term "cured" means that the polymeric diffusion matrix contains little or no excess water used in forming the matrix. As shown below, the diffusion matrix is formed by mixing together the glycerol, polyvinylalcohol, the water-soluble polymer with hydration sites and water to obtain a homogeneous mixture which is cast into sheets of the matrix. In order to allow casting of the mixture, it is sometimes necessary to use an excess amount of water. Immediately after casting, the polymeric matrix is in an "uncured" state. The excess water may then be permitted to evaporate. When substantially all of the excess water has evaporated, the polymeric matrix is in a "cured" state. As a result of the evaporation of the water, which generally requires from about 1 to about 18 hours, the thickness of the diffusion matrix is reduced, or a "collapsed" matrix is obtained.

In the uncured matrix, the glycerol is present in an amount of from 2 to 60%, preferably 2 to 20%, by weight. However, when trinitroglycerol is the drug to be applied, the amount of glycerol should be within the range of from 35 to 60%. Preferably, the glycerol has a mimimum specific gravity of 1.23 g/ml.

The polyvinylalcohol is present in the uncured matrix in an amount of from 2 to 15%, preferably from 4 to 9% by weight. Preferably, the polyvinylalcohol has a molecular weight of at least 70,000. Most preferably, the molecular weight is from 100,000 to 150,000.

The water-soluble polymer with hydration sites is present in the uncured matrix in an amount of from 2 to 10%, preferably from 2 to 5%, by weight. In a preferred embodiment, polyvinylpyrrolidone is used as the water-soluble polymer. The molecular weight for the polyvinylpyrrolidone should be selected to maintain water solubility. In general, this molecular weight should be within the range of from 20,000 to 60,000, preferably from 35,000 to 50,000. The polyvinylpyrrolidone may be replaced by other ingredients which permit sustained release. For example, agar in an amount of from 2% to 6% by weight may be used.

The balance of the matrix comprises essentially water.

When the matrix is uncured, it preferably comprises from 2 to 20% of glycerol, from 4 to 12% of polyvinylalcohol, from 2 to 8% of the water-soluble polymer having hydration sites, and the balance water.

In its cured state, the polymeric diffusion matrix comprises from 2 to 55%, preferably from 4 to 35% of glycerol, from 4 to 30%, preferably from 8 to 20% of polyvinylalcohol; from 2 to 20%, preferably from 4 to 10% of a water-soluble polymer having hydration sites, preferably polyvinylpyrrolidone, and the balance water, all percentages being by weight. The molecular weight ranges for the polyvinylalcohol and polyvinylpyrrolidone are the same for cured and uncured diffusion matrices. The cured matrix has a density of about 1.2 g/ml. It is noted that the weight ratio of glycerol to water in the cured matrix is 0.6—1.8:1, preferably 1:1. The cured matrix shows little swelling when immersed in water and will not dissolve in water at room temperature. However, if the water is heated to boiling, the diffusion matrix will dissolve.

At least one drug may be dispersed throughout the diffusion matrix. The type of drug which may be dispersed in the diffusion matrix of the present invention includes any drug which is capable of being transdermally administered to a patient. With the sustained release of the drug at a relatively steady rate over a prolonged period, typically 24 hours, the patient is provided with the benefit of a steady application of the drug over the prolonged period. Examples of drugs which are suitable for inclusion in the diffusion matrix of the present invention include the following: alpha-[1-(methylamino)-ethyl]-benzene methanol, which is useful as an adrenergic (bronchodilator); N-phenyl-N-[1-(2-phenylethyl)-4-piperidinyl propanamide, useful as a narcotic analgesic; 6-chloro-3,4-dihydro-2H-1,2,4-benzothiadiazine-7-sulphonamide 1,1-dioxide, useful as a diuretic; 2-diphenylmethoxy-N,N-dimethyl-ethanamine, useful as an antihistamine; and an estrogen. Other useful drugs include: anti-microbial agents such as penicillin, tetracycline, oxytetracycline, chlortetracycline, chloramphenicol, and sulphonamides; sedatives and hypnotics such as pentabarbital sodium, phenobarbital, secobarbital sodium, codeine, (-bromoisovaleryl)urea, carbromal, and sodium phenobarbital, psychic energizers such as 3-(2-aminopropyl)indole acetate and 3-(2-aminobutyl) indole acetate; tranquilizers such as reserpine, chlorpromazine hydrochloride, and thiopropazate hydrochloride; hormones such as adreno-cortisosteroids, for example, 6-methylprednisolone; androgenic steroids, for example, methylesto-sterone, and fluoxymesterone; estrogenic steroids, for example, estrone, estradiol and ethinyl estradiol; progestational steroids, for example, 17-hydroxyprogesterone acetate, medroxyprogesterone acetate, 19-norprogesterone, and norethindrone; and thyroxine; antipyretics such as aspirin, salicylamide, and sodium salicylate; morphine and other narcotic analgesics; antidiabetics, e.g. insulin; antispasmodics such as atropine, methscopolamine bromide, methscopolamine bromide with phenobarbital; antimalarials such as the 4-aminoquinolines, 9-aminoquinolines, and pyrimethamine; and nutritional agents such as vitamins, essential amino acids, and essential fats. The above listing of drugs is merely exemplary of the transdermally applicable drugs. It is contemplated that any drug which may be transdermally applied is suitable for use as the drug to be applied via the diffusion matrix in the present device.

It will be appreciated that the drug may be added to the above mixture not only in the form of the pure chemical compound, but also in admixture with other drugs which may be transdermally applied or with other ingredients which are not incompatible with the desired objective of transdermally administering the drug to a patient. Thus, simple pharmacologically acceptable derivatives of the drugs such as ethers, esters, amides, acetals, salts and the like may be used. In some cases such derivatives may actually be preferred.

The amount of the drug dispersed in the diffusion matrix can be varied in accordance with the desired dosage and the length of time the matrix is to remain on the skin. However, the amount of the drug included in the matrix should generally be in excess of the amount which is to be delivered to the patient. If the diffusion matrix is to be used for 24 hours, an approximate 10-fold excess of the drug should be included. For example, if it is desired to apply about 5 mg of trinitroglycerol to a patent over 24 hours, a roughly ten-fold excess of the trinitroglycerol should be included in the diffusion matrix. Accordingly, from 40 to 60 mg is considered a preferred amount to provide a 5 mg release of trinitroglycerol over a 24 hour period. Quite obviously, the optimum amount that should be included in the diffusion matrix will vary according to factors such as the period of release of the drug.

In a preferred embodiment, there is used trinitroglycerol or 1,2,3-propanetriol trinitrate, which is useful in coronary medicine as a vasodilator. It is preferred to add the trinitroglycerol in the form of a lactose triurate, in view of the danger of explosion or trinitroglycerol. In addition, the ratio of lactose triturate to the water and glycerol should avoid proportions where the trinitroglycerol may separate and raise an explosion hazard. A preferred lactose triturate is a composition comprising 10% of nitroglycerin and 90% of beta-lactose.

In forming the trinitroglycerol-containing matrix, excess water is not required. Hence, this matrix comprises from 35 to 60%, preferably from 45 to 55% of glycerol; from 2 to 15%, preferably from 4 to

3

**O 013 606**

9% of polyvinylalcohol; and from 2 to 10%, preferably from 2 to 5% of polyvinylpyrrolidone, the balance being essentially water and all percentages being by weight. We have found that the amount of water evaporated from the uncured matrix is negligible, hence, the higher percentage for the glycerol. For this matrix, the weight ratio of glycerol to total polymers is usually greater than 1, preferably from 1.4 to 15:1. The polyvinylpyrrolidone may be replaced by other water-soluble polymers, in particular agar.

The amount of trinitroglycerol which should be used is based upon a desired delivery of about 5 mg per patient over a 24 hour period. The diffusion matrix drug delivery system of the present invention to deliver the 5 mg in the 24 hour period should contain about 40 to 60 mg of the trinitroglycerol. To reach this objective, the concentration of the trinitroglycerol in the diffusion matrix and the area of the diffusion matrix are factors to consider. In accordance with a preferred aspect of the present invention, from 0.1 to 4.0% by weight of trinitroglycerol is included in the diffusion matrix. In a preferred aspect of the present invention, 80 ml of the solution is mixed with 20 gm of lactose triturate, and this mixture is mechanically stirred until it is homogeneous. The resultant homogeneous mixture is poured into forms preferably made of glass or stainless steel, these forms or templates producing a diffusion matrix having a thickness of 3 to 4 mm, in accordance with a preferred aspect of the present invention. This diffusion matrix is either cast or cut into pieces of the desired size. In a preferred aspect, squares of one inch (2.5 cm) on each side, or 6.5 cm$^2$, have been prepared for each of application to the patient.

The following methods have been found convenient for preparing the diffusion matrix of the present invention.

In a first method, the matrix is formed at atmospheric pressure. Water and glycerol are first mixed together. Since it has been found that alkaline mixtures have relatively poor stability, the pH of the mixture is adjusted so that it is either neutral or slightly acidic, i.e., the pH ranging from 6.5 to 7.0. In a preferred embodiment, the pH is adjusted to within the above-mentioned range by adding sodium citrate and citric acid to the mixture.

The polyvinylalcohol and polyvinylpyrrolidone are then added to the glycerol-water mixture at room temperature, with agitation. The mixture is heated to a temperature within the range of from 90 to 95°C at atmospheric pressure to extend the polymers. The mixture is held at this temperature for about one hour. If desired, the mixture may be maintained at this temperature for a period of about 48 hours prior to the addition of the drug. That is, the mixture is stable for a period of about 48 hours and may be kept for such a period before being mixed with the drug to be delivered to the patient. Thereafter, the mixture is cooled to 80°C and stirred for an additional hour to remove bubbles therefrom. The drug to be applied to the patient is then added to the mixture, with thorough agitation. Once a homogeneous mixture of the polymer solution and drug is obtained, the mixture is ready to be cast into sheets of the drug-containing diffusion matrix. In a preferred embodiment, the drug may be dissolved by agitation in a suitable solvent such as glycerin and water. The solution obtained in this way can be maintained at room temperature for prolonged periods without deterioration.

In a second method, water and glycerol are mixed, with the pH of the mixture adjusted to a desired value by adding suitable amounts of sodium citrate and citric acid. Thereafter, the polyvinylalcohol and polyvinylpyrrolidone are added. The resulting mixture is then heated to a temperature of about 120°C at a pressure of about 2 atmospheres absolute. The temperature is maintained for about 1 hour without any mechanical agitation. In a preferred embodiment, the heating may be performed in an autoclave. Since bubbles are not formed when the heating is conducted in an autoclave, such a procedure is preferred. Thereafter, the temperature is lowered to 20 to 80°C whereupon the drug to be applied to the patient is added. After the drug has been homogeneously dispersed in the liquid mixture, the mixture is poured into moulds to form sheets of the drug-containing diffusion matrix.

In the above methods and for the case of nitroglycerin and other drugs having similar limitations, the drug must be added and mixed thoroughly when the polymer mixture is in the liquid state. Furthermore, the mixture should be cast within about 30 minutes after the drug has been introduced into the polymer solution. This is important in order to avoid the setting of the polymer solution prior to casting.

The temperature at which the drug is to be added to the matrix solution depends on the stability of the drug. For example, nitroglycerin begins to decompose at a temperature of above about 50°C. Accordingly, in preparing a nitroglycerin-containing diffusion matrix, the matrix solution mixture is cooled to about 50°C, whereupon the nitroglycerin is added. The drug-containing diffusion solution is then cast into moulds to form sheets of the final product. In addition, for nitroglycerin, the pH of the solution mixture should be kept slightly acidic, i.e. between 6.5 and 7.0, since nitroglycerin is stabilized within this pH range.

Dodecyl alcohol of sorbitan (Tween-20) or other detergents may be added in an amount of 0.1 to 10% by weight, based on the matrix, as a dispersing agent, if desired.

For drugs that are alcohol-soluble, it may be desirable to add ethanol or isopropanol in an amount of from 2 to 40% by weight, based on the matrix, in the initial mixture of glycerol and water, to facilitate the preparation of a diffusion matrix for such alcohol-soluble drugs. In addition, ethanol and isopropanol, when added to the initial mixture, will provide a "collapsed" diffusion matrix, i.e. as the

4

ethanol and isopropanol evaporate the diffusion matrix produced in accordance with the present invention will "collapse".

An absorption facilitator to ensure skin penetration such as dimethylsulphoxide, decylmethylsulphoxide, or other penetration enhancers may be added.

If it is desired to increase the effective lifetime of the diffusion matrix, a drug reservoir may also be attached to the diffusion matrix. The diffusion matrix may also be used to help with local vasodilation to assist in the solution of physiological problems resulting from local circulatory difficiencies, for example, to promote circulation in the extremities of a geriatric patient.

The present drug delivery device comprises the drug-containing diffusion matrix and means for fastening the matrix to the skin of a patient. Such means can take various forms, such as an occlusive backing layer forming a kind of "bandage" with the diffusion matrix being held against the skin of a patient being treated. A polyethylene or polyethylene terephthalate tape is contemplated as one form of occlusive layer in accordance with the present invention. It can also take the form of an elastic band, such as a cloth band, a rubbery band or other material. Here, the diffusion matrix is placed directly on the skin and held in place by such elastic band which typically will be placed over the arm or wrist of the patient. An intermediate adhesive layer between the diffusion matrix and the skin capable of permitting the transdermal application of the drug can also be used.

As a preferred embodiment in the packaging of the present matrix, the drug-containing diffusion matrix is placed in a cavity provided in an inert backing material. Useful backing materials include metal foils such as aluminium foil, polyolefins such as polyethylene and polypropylene, polyesters such as polyethylene terephthalate or polyamides. The drug-containing diffusion matrix can be poured in its molten state into the cavity and permitted to cool. An adhesive layer is provided on the backing material surrounding the cavity. To prefent air from coming into contact with the matrix, the adhesive layer and the matrix are sealed with a release layer. To use the device, the patient peels off the release layer and places the device in intimate contact with his skin. The exposed adhesive layer secures the device to the patient. Since a concentration gradient exists in a plane normal to the surface of the matrix and the patient's skin, this condition facilitates the diffusion of the drug through the matrix into the patient's body. Thus, there is provided a device whereby a drug is delivered transdermally to a patient at a steady rate over a prolonged period of time.

Reference is now made to the accompanying drawings, in which:

Figure 1 shows a plan view of a bandage having incorporated therein the drug-containing polymeric diffusion matrix of the present invention; and

Figure 2 illustrates the cross-sectional view along line 2—2' in Figure 1.

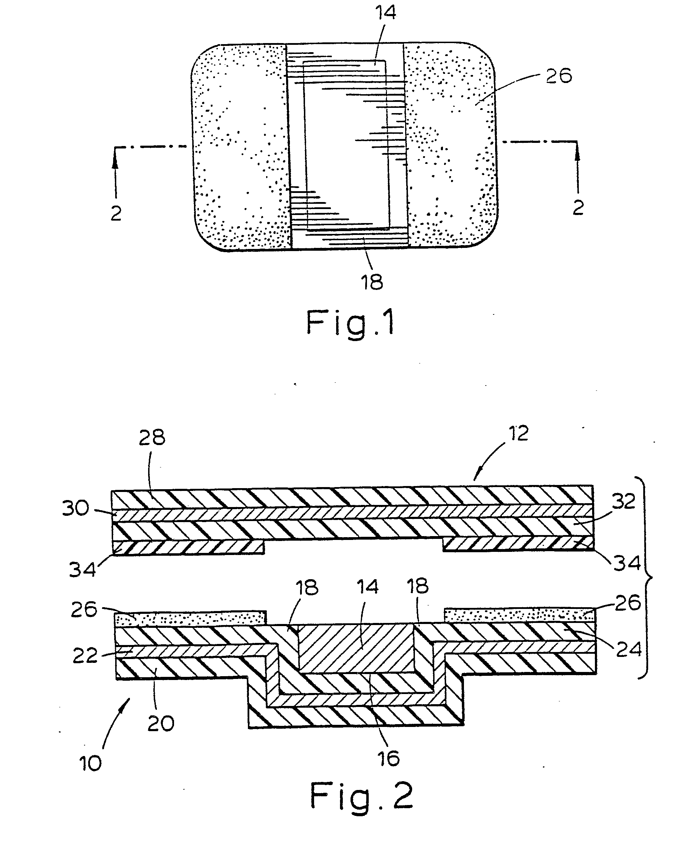

The construction of a preferred embodiment for the packaging of the present invention is shown in further detail in Figures 1 and 2. As illustrated in the figures, the package comprises a bandage having a cover layer 12 and a backing member 10. The diffusion matrix 14 having a drug (e.g. trinitroglycerol) dispersed therein is placed in cavity 16 in the backing member 10. The diffusion matrix may be poured in its molten state into the cavity 16 in the backing member 10 and permitted to cure. Alternatively, the molten polymeric mixture (with or without a drug) is cast to form a thin sheet which is cut, after curing, into smaller sheets to fit the particular application of the matrix. Individual smaller sheets may then be placed in the cavity 16 in the backing member 10. The area 18 surrounding the matrix in the backing member 10 is heat-sealed to prevent the matrix from being removed from the backing member. The backing member 10 is formed of a laminate comprising an outer layer 20 made of a polyester, such as polyethylene terephthalate, an intermediate layer 22 made of a metallic foil, e.g. aluminium foil, and an inner layer 24 made of an ionomer such as Surlyn®.

A layer of pressure sensitive adhesive 26 is provided on the surface of the inner layer surrounding the heat sealed portion. It is noted that the adhesive does not cover the matrix.

The matrix is prevented from coming into contact with the atmosphere by placing the cover layer 12 thereon, which seals the matrix. The cover layer is also formed of a laminate having the same construction as the backing layer, i.e. an outer layer 28 made of a polyester, e.g. polyethylene terephthalate; an intermediate layer 30 made of a metallic foil, e.g. aluminium foil; and an inner layer 32 of an ionomer, e.g. Surlyn®. The surface of the inner layer coming into contact with the pressure sensitive adhesive 26 on the backing member 10 is coated with a release layer 34 to permit easy removal of the cover layer.

To apply the drug to the patient, the cover layer is peeled off. The exposed matrix is then taped onto a suitable portion of the patient's body, e.g. arm or wrist, to allow the drug to diffuse thereinto.

In the preferred embodiment wherein trinitroglycerol is dispersed in the polymeric diffusion matrix, the molten matrix is cast into cavities provided in the backing member. The matrix is permitted to cure for a short period (e.g. 10 minutes to one hour) and is sealed by placing the cover layer over the backing member.

For a matrix in an uncured state, the water-soluble polymer is present in an amount of from 2 to 10%, preferably from 2 to 8% by weight. For polyvinylpyrrolidone, which is a preferred water-soluble polymer, it has a molecular weight of from 20,000 to 60,000, preferably from 35,000 to 50,000. The polyvinylalcohol is present in an amount of from 2 to 15% preferably from 4 to 12% by weight. The polyvinylalcohol has a molecular weight of from 100,000 to 150,000, preferably from 120,000 to 135,000. The glycerol is present in an amount of from 2 to 20%, preferably from 2 to 18% by weight. Preferably, the glycerol is a 96% aqueous glycerol solution.

As a preferred embodiment, there is provided a polymeric diffusion matrix which comprises in its uncured state and on a weight basis: about 10.5% of polyvinylalcohol (molecular weight 126,000); about 6% of polyvinylpyrrolidone (molecular weight 40,000); about 15% of glycerol; and the balance water.

The relative weight amounts of polyvinylalcohol to polyvinylpyrrolidone that have been considered range from 3:1 to 1:1. In actual practice, however, at a range of about 3:1, less than optimum results are obtained with the matrix swelling to an unacceptable degree, and at the ratio of 1:1 the matrix tends toward being soft and sticky. Thus, in accordance with a preverred aspect of the present invention, we have found that a weight range of polyvinylalcohol to polyvinylpyrrolidone should be between 2:1 and 3:2. The weight ratio of glycerol to total polymers for the matrix is usually less than 1, preferably 0.5—1:1.

The method of administration of this invention is suitable also for adaptation to buccal and especially to sublingual administration. Because of the much higher rate of absorption through the mucosa by that route, much shorter periods of administration are required.

The matrices of the present invention are illustrated in the following Examples. Since the Examples are for illustrative purposes, they should not be construed as limiting.

Example I

45 ml of glycerol and 45 ml of water together with 1% by weight of sodium citrate are mixed together and the pH is adjusted to 7 through addition of citric acid. This mixture is heated to 90°C; after reaching at least 70°C there are slowly added 7 g of polyvinyl alcohol (PVA 100% hydrolyzed, molecular weight 115,000) and 5 g of polyvinylpyrrolidone (mw 40,000). The mixture is stirred at 90°C until solution is effected, which may take about 10 minutes, it being appreciated that with larger quantities, a considerably longer period of time may be needed. 80 ml of this solution is then mixed with 20 g of lactose triturate (10% of nitroglycerin and 90% of lactose), this mixture then being mechanically stirred until homogenous. The homogenous mixture is then poured into forms made of glass or stainless steel which serve as templates to produce a diffusion matrix having a thickness of about 3 to 4 mm. This diffusion matrix is then cut into square pieces of about 1 inch (2.5 cm) on each side, i.e. to provide a total surface area of about 6.5 cm².

Example II

Example I is repeated with the exception that 3 g of agar is used instead of the polyvinylpyrrolidone. Also included in the mixture is 1% by weight of calcium chloride.

Example III

The diffusion matrix of Example I is applied to a patient by placing it against the wrist, shoulder or other sites of the patient.

Example IV

The diffusion matrix of Example I is applied to a patient by first attaching the diffusion matrix to a polyethylene terephthalate or polyethylene backing layer. This occlusive backing layer is provided with an adhesive whereby the diffusion matrix is held in contact with the skin as part of this "bandage".

Examples V—X

By substituting an appropriate amount of the following chemicals, in place of the lactose triturate, and otherwise following the procedure of Example I, diffusion matrices are obtained:

| Example | Compound | Use |
|---------|----------|-----|
| V | Alpha-[1-(methylamino)-ethyl]-benzene methanol | adrenergic (broncho-dilator) |
| VI | N-phenyl-N-[1-(2-phenylethyl)-4-piperidinyl]-propamide | narcotic analgesic |
| VII | 6-chloro-3,4-dihydro-2H-1,2,4-benzothiadiazine-7-sulphonamide-1,1-dioxide | diuretic |
| VIII | 2-diphenylmethoxy-N,N-dimethyl-ethanamine | antihistamine |
| IX | estra-1,3,5(10)triene-3-,17beta-diol | estrogenic |
| X | 5-ethyl-5-phenyl-2,4,6(1H,3H,5H)-pyrimidinetrione | anticonvulsant, hypnotic, sedative |

Example XI

948 g of 96% glycerol and 644 g of water are mixed together. 27 g of sodium citrate, 159 g of polyvinyl alcohol (molecular weight 115,000) and 93 g of polyvinylpyrrolidone (molecular weight 40,000) are dissolved in the glycerol/water mixture by continuous stirring and maintaining at a temperature of about 90°C.

In a separate container, 600 g of nitroglycerin triturate (10% nitroglycerin and 90% of lactose) are dissolved in 315 g of glycerol and 214 g of water with agitation at room temperature.

When the polymers have gone into solution, the nitroglycerin dispersion is poured therein. The mixture is mixed thoroughly at a temperature range of between 50 and 55°C to form a homogeneous mixture. The container is kept covered.

The homogeneous mixture is poured into forms made of glass or stainless steel which serve as templates to produce a drug-containing diffusion matrix having a thickness of about 3 to 4 mm. This diffusion matrix is then cut into square pieces of about 1 inch (2.5 cm) on each side, i.e. to provide a total surface area of about 6.5 cm$^2$.

Example XII

Male dogs are anesthetized with sodium pentothal. Through surgical incisions, catheters are positioned in the femoral veins of each hind leg and the abdominal aorta. Flow gauges are placed on the internal iliacs of both hind limbs. On a well-shaved area of the medial surface of the left thigh, a nitroglycerin-containing polymer matrix obtained in Example 1 is taped in place and remains undisturbed for 4 hours. The right hind limb receives no matrix or treatment of any kind. After application of the matrix, blood samples (5 ml) are taken from the catheters in each of the femoral veins and from that in the abdominal aorta at 15, 30, 60, 120, 180 and 240 minutes. Once drawn, the blood samples are put in ice, centrifuged (for 10 minutes) at 0°C, and 2 ml of plasma are transferred to a silanized (with an alkylated silicone oil) glass tube. To each tube, 5 ml of *n*-pentane is also added and the nitroglycerin is extracted for 1 hour with gentle shaking at 0°C. The pentane phase is then transferred to a 5 ml capacity Reaction-Vial and evaporated to near dryness. The residue is then dissolved in 30 microlitres of benzene containing 2 nanograms of para-nitro-anisole used as the external standard. 1.0 to 50.0 microlitres of this solution are then injected for nitroglycerin quantitation using GLC-Electron Capture Detection. (A Hewlett-Packard 4610A Gas chromatograph equipped with a $^{63}$Ni-electron capture detector is used.) Separation is achieved on a 4 foot (120 cm)×3 mm I.D. glass column packed with 10% SE-30 on 100/120 mesh Gas-Chrom Q (Trade Mark). The column is maintained at 140°C while the injection-port temperature is 170°C and the detector temperature is 220°C. A nitroglycerin calibration curve is constructed from the analyses of nitroglycerin spiked blank-plasma.

The results from the above test runs, summarized in Table 1, show dramatically that nitroglycerin is absorbed transepidermally from the matrix over the entire 4 hour period. Also, the levels attained in the venous blood draining the limb containing the matrix are grossly proportional to the matrix surface area in contact with the skin.

From the results of the studies here discussed, it is evident that transepidermal nitroglycerin absorption has occurred from the matrix to blood.

The nitroglycerin absorption rate appears to be fairly constant from 30—240 minutes as depicted by the essentially non-varying arterial nitroglycerin plasma levels.

TABLE 1

| Matrix size— Study no. Sample | 5.0 cm×7.5 cm 1 nanograms | 5.0 cm×2.5 cm 2 nitroglycerin per ml | 2.5 cm×2.5 cm 3 plasma |
|---|---|---|---|
| Arterial —15 min | 0.68 | 0.14 | 0.27 |
| „ —30 min | 0.57 | 0.15 | 0.53 |
| „ —60 min | 0.73 | 0.15 | — |
| „ —120 min | 0.85 | 0.49 | 0.36 |
| „ —180 min | 1.29 | 0.68 | 0.50 |
| „ —240 min | 1.26 | 0.21 | 0.30 |
| Experimental venous—15 min | 0.95 | 5.70 | 0.40 |
| „ —30 min | 0.51 | 8.31 | 0.32 |
| „ —60 min | 15.3 | 11.4 | 0.52 |
| „ —120 min | 26.9 | 7.63 | 0.75 |
| „ —180 min | 32.9 | 13.7 | 0.57 |
| „ —240 min | 32.0 | 5.55 | 0.23 |
| Control venous—15 min | 0.44 | 9.18 | 0.09 |
| „ —30 min | 0.61 | 21.6 | 0.15 |
| „ —60 min | 7.40 | 4.51 | 0.28 |
| „ —120 min | 2.33 | 13.0 | 0.42 |
| „ —180 min | 9.87 | 14.5 | 0.39 |
| „ —240 min | 13.9 | 4.10 | 0.23 |

Example XIII

Five male mongrel dogs, free of disease, are anethetized with sodium pentabarbital. Under a septic surgical procedure, a catheter is inserted into the right artrium via the jugular vein for the removal of blood samples from the right heart. An arterial catheter is placed in the right carotid artery for the continuous recording of arterial blood pressure. Both catheters are exteriorized at the back of the neck.

The animals are allowed to recover from the anesthetic and are studied 24 hours later in the fasted, conscious state while resting comfortably in a supporting harness.

Each animal is allowed to become familiar with the laboratory surroundings and when completely acclimated, a 20 ml reference blood sample is obtained from the right heart catheter. A 2.5 cm×2.5 cm square of the nitroglycerin containing polymer matrix obtained in Example I is then applied to a well shaved area of the right lateral chest wall. The matrix is held securely in place with surgical tape. After application of the polymer matrix, 5.0 ml blood samples are obtained at: 15 min, 30 min, 45 min, 1 hr, 2 hr, 3 hr, 4 hr, 5 hr, 6 hr, 7 hr, 8 hr, 9 hr, 10 hr, 11 hr, 12 hr, 14 hr, 16 hr, 18 hr, 20 hr, 22 hr and 24 hr. The animals are conscious and unrestrained during the entire 24 hour period of sampling. At no time do the animals display any unfavourable effects due to the transcutaneous administration of nitroglycerin.

Immediately after drawing, the blood samples are put in ice and transferred to a walk-in refrigerator and centrifuged for 10 minutes at 0°C. A 2 ml aliquot of plasma is taken from each specimen and transferred to individual silanized (with an alkylated silicone oil) glass tubes. A 5 ml volume of n-pentane is added to each tube and the nitroglycerin is extracted for 60 minutes with gentle shaking at 0°C. The pentane phase is transferred to a 5 ml capacity Reaction-Vial and evaporated to near dryness. The residue is dissolved in 30 microlitres of benzene containing 2 nanograms of para-nitro-anisole used as the external standard. A 1.0 to 5.0 microlitre aliquot of this solution is injected for nitroglycerin quantitation using GLC-Electron Capture Detection (Hewlett-Packard 4610A Gas Chromatograph equipped with a $^{63}$Ni-electron capture detector). Separation is achieved on a 4 foot (120 cm×3 mm I.D. glass column packed with 10% SE-30 on 100/120 mesh Gas-Chrom Q (Trade Mark). The column is maintained at 140°C while the injection-port temperature is 170°C and the detector temperature is 220°C. A nitroglycerin calibration curve is constructed from the analyses of nitroglycerin-spiked blank plasma.

Table 2 summarizes the plasma nitroglycerin data from the dogs. At each time point the mean ± the standard deviation is listed in the Table.

TABLE 2

| Hours post application | ng Nitroglycerin/ml plasma | | | | | | |
|---|---|---|---|---|---|---|---|
| | Dog 1 | Dog 2 | Dog 3 | Dog 4 | Dog 5 | 5 Dogs | +/−S.D. |
| 0.25 | 0.11 | 0.39 | — | 0.37 | — | 0.29 | 0.16 |
| 0.50 | 0.08 | 0.28 | 0.02 | 0.16 | 0.16 | 0.14 | 0.098 |
| 0.75 | 0.08 | 0.29 | 0.02 | 0.14 | 0.19 | 0.14 | 0.10 |
| 1.00 | 0.23 | 0.19 | 0.15 | 0.10 | 0.36 | 0.21 | 0.099 |
| 2.00 | 0.22 | 0.57 | 0.02 | 0.22 | 0.27 | 0.26 | 0.20 |
| 3.00 | 2.06 | 0.38 | 0.04 | 0.88 | 0.17 | 0.71 | 0.82 |
| 4.00 | 0.52 | 0.81 | 0.11 | 0.28 | 0.26 | 0.40 | 0.27 |
| 5.00 | 0.22 | 1.00 | 0.28 | 0.17 | 0.11 | 0.36 | 0.37 |
| 6.00 | 0.23 | 0.63 | 0.24 | 0.55 | 0.88 | 0.51 | 0.28 |
| 7.00 | 0.93 | 0.70 | 0.45 | 0.34 | 1.23 | 0.73 | 0.36 |
| 8.00 | 0.16 | 2.39 | 0.70 | 0.42 | 0.45 | 0.82 | 0.90 |
| 9.00 | 0.22 | 0.59 | 0.32 | 0.34 | 0.10 | 0.31 | 0.18 |
| 10.00 | 0.11 | 0.83 | 0.59 | 0.31 | 0.66 | 0.50 | 0.29 |
| 11.00 | 0.07 | 0.77 | 0.13 | 0.35 | 0.50 | 0.36 | 0.28 |
| 12.00 | 0.35 | 0.55 | 0.37 | 0.29 | 0.12 | 0.34 | 0.15 |
| 14.00 | 0.04 | 0.39 | 0.18 | 0.17 | 0.20 | 0.20 | 0.13 |
| 16.00 | 0.07 | 0.41 | 0.28 | 0.57 | 0.32 | 0.38 | 0.17 |
| 18.00 | 0.26 | 1.17 | 0.32 | 0.29 | 0.39 | 0.49 | 0.39 |
| 20.00 | 0.34 | 0.41 | 0.52 | 0.33 | 0.24 | 0.31 | 0.11 |
| 22.00 | 0.20 | 1.11 | 0.66 | — | — | 0.66 | 0.46 |
| 24.00 | 0.27 | 0.43 | — | — | — | 0.35 | 0.11 |

From these results, it is evident that transcutaneous nitroglycerin absorption does occur, and does so at a constant and continuous rate so as to achieve a plateau plasma nitroglycerin level ranging from about average values of 0.3—0.6 ng nitroglycerin/ml plasma. The data also show that the temporal limits of the nitroglycerin matrix have not been exceeded, or for that matter, have not been approached during the 24 hour experimental period. In each case, the apparent plateau nitroglycerin level shows no evidence of decreasing, either before or at the 24 hour experimental time limit.

**Claims for the Contracting States: BE, CH, DE, FR, GB, IT, LU, NL, SE**

1. A drug delivery system for sustained release of a drug, said system comprising:

(A) a polymeric diffusion matrix comprising from 2 to 60% of glycerol, from 2 to 15% of polyvinylalcohol, from 2 to 10% of a water-soluble polymer with hydration sites, and the balance water, the percentages being by weight, and
(B) a therapeutically effective amount of at least one drug suitable for transdermal delivery to a patient, said drug component (B) being dispersed in said matrix (A) or being deposited in a drug reservoir attached to said matrix (A).

2. A drug delivery system as claimed in claim 1, wherein said drug is trinitroglycerol.

3. A drug delivery system as claimed in claim 1, wherein said drug is an antibiotic, a local anesthetic, an analgesic, a fungicide, a bactericide, or an antimicoplasma.

4. A drug delivery system as claimed in any of claims 1 to 3, wherein the matrix is cured and comprises from 2 to 55% of glycerol, from about 4 to 30% of polyvinylalcohol, from 2 to 20% of the water-soluble polymer having hydration sites, and the balance water.

5. A drug delivery system as claimed in any of claims 1 to 4, wherein the water-soluble polymer is polyvinylpyrrolidone, agar, agarose or a water-soluble cellulose derivative.

6. A drug delivery system as claimed in any one of claims 1 to 5, wherein the polyvinylalcohol has a molecular weight of from 100,000 to 150,000, and the polyvinylpyrrolidone, when present as the water-soluble polymer, has a molecular weight of from 20,000 to 60,000.

7. A drug delivery device comprising a drug delivery system constituted by a polymeric diffusion matrix (14) together with a therapeutically effective amount of at least one drug as claimed in any one of claims 1 to 6, and means (10) for securing said matrix provided with said drug to the skin of a patient.

8. A method of making a drug delivery system which method comprises mixing, in an uncured state and on a weight basis, from 2 to 60% of glycerol, from 2 to 15% of polyvinylalcohol, from 2 to 10% of a water-soluble polymer having hydration sites, and the balance water, heating the resulting mixture and casting the mixture to form a polymer diffusion matrix, wherein a therapeutically effective amount of at least one drug suitable for transdermal application to a patient is added to and dispersed in the mixture or deposited as a reservoir attached to the surface of said diffusion matrix.

9. A method as claimed in claim 8, comprising the following steps:

(a) mixing the glycerol with water;

(b) dissolving the polyvinylalcohol and the water-soluble polymer having hydration sites in the mixture of (a) by stirring and heating to from 90 to 95°C; and

(c) casting the mixture to form sheets of the diffusion matrix,

said drug being added to and dispersed in the mixture resulting from step (b), or deposited on the surface of said matrix.

10. A method as claimed in claim 9, including the additional step of curing the matrix obtained in (c) to produce a cured matrix comprising, on a weight basis, from 2 to 55% of glycerol, from 4 to 30% of polyvinylalcohol, from 2 to 20% of the water-soluble polymer having hydration sites, and the balance water.

11. A drug delivery system as claimed in any of claims 1 to 6, or made by a method as claimed in any of claims 8 to 10, or a drug delivery device as claimed in claim 7, for use in the transdermal delivery to a patient of at least one drug.

## Claims for the Contracting State: AT

1. A method of making a drug delivery system which method comprises mixing, in an uncured state and on a weight basis, from 2 to 60% of glycerol, from 2 to 15% of polyvinylalcohol, from 2 to 10% of a water-soluble polymer having hydration sites, and the balance water, heating the resulting mixture and casting the mixture to form a polymer diffusion matrix, wherein a therapeutically effective amount of at least one drug suitable for transdermal application to a patient is added to and dispersed in the mixture or deposited as a reservoir attached to the surface of said diffusion matrix.

2. A method as claimed in claim 1, comprising the following steps:

(a) mixing the glycerol with water;

(b) dissolving the polyvinylalcohol and the water-soluble polymer having hydration sites in the mixture of (a) by stirring and heating to from 90 to 95°C; and

(c) casting the mixture to form sheets of the diffusion matrix,

said drug being added to and dispersed in the mixture resulting from step (b), or deposited on the surface of said matrix.

3. A method as claimed in claim 1 or 2, wherein the water-soluble polymer is agar, agarose, polyvinylpyrrolidone or a water-soluble cellulose derivative.

4. A method as claimed in claim 3, including the additional step of curing the matrix obtained in (c) to produce a cured matrix comprising, on a weight basis, from 2 to 55% of glycerol, from 4 to 30% of polyvinylalcohol, from 2 to 20% of the water-soluble polymer having hydration sites, and the balance water.

5. A method as claimed in any one of claims 1 to 4, wherein the drug is trinitroglycerol.

6. A method as claimed in claim 5, wherein said trinitroglycerol is dispersed in the mixture of (b) when the temperature of the mixture has been lowered to from 50 to 55°C.

## Patentansprüche für die Vertragsstaat: BE, CH/LI, DE, FR, GB, IT, LU, NL, SE

1. Wirkstoffabgabesystem zur fortgesetzten Abgabe eines Medikaments, umfassend

a) eine polymere Diffusionsmatrix, die 2 bis 60% Glycerin, 2 bis 15% Polyvinylalkohol, 2 bis 10% eines wasserlöslichen Polymers mit Hydratisierungs-Zentren und als Rest Wasser enthält, wobei die Prozentzahlen sich auf das Gewicht beziehen, und

b) eine therapeutisch wirksame Menge wenigstens eines Medikaments, das für eine transdermale Verabreichung an einen Patienten geeignet ist,

wobei die Medikament-Komponente (B) in der Matrix (A) dispergiert ist oder in einem an der Matrix (A) angebrachten Medikamenten-Reservoir deponiert ist.

2. Wirkstoffabgabesystem nach Anspruch 1, dadurch gekennzeichnet, daß das Medikament Trinitroglycerin ist.

3. Wirkstoffabgabesystem nach Anspruch 1, dadurch gekennzeichnet, daß das Medikament ein Antibiotikum, ein Lokalanästhetikum, ein Analgetikum, ein Fungizid, ein Bakterizid oder ein Antimycoplasma ist.

4. Wirkstoffabgabesystem nach einem der Ansprüche 1 bis 3, dadurch gekennzeichnet, daß die Matrix gehärtet ist und 2 bis 55% Glycerin, 4 bis 30% Polyvinylalkohol, 2 bis 20% des wasserlöslichen Polymers mit Hydratisierungs-Zentren und als Rest Wasser enthält.

5. Wirkstoffabgabesystem nach einem der Ansprüche 1 bis 4, dadurch gekennzeichnet, daß das wasserlösliche Polymer Polyvinylpyrrolidon, Agar, Agarose oder ein wasserlösliches Cellulose-Derivat ist.

6. Wirkstoffabgabesystem nach einem der Ansprüche 1 bis 5, dadurch gekennzeichnet, daß der Polyvinylalkohol ein Molekulargewicht von 100 000 bis 150 000 besitzt und das Polyvinylpyrrolidon, wenn es in dem Wasserlöslichen Polymer vorhanden ist, ein Molekulargewicht von 20 000 bis 60 000 besitzt.

7. Wirkstoffabgabevorrichtung, enthaltend ein Wirkstoffabgabesystem gebildet aus einer polymeren Diffusionsmatrix (14) zusammen mit einer therapeutisch wirksamen Menge wenigstens eines Medikaments nach einem der Ansprüche 1 bis 6 und ein Mittel (10) zur Befestigung der mit dem Medikament versehenen Matrix auf der Haut des Patienten.

8. Verfahren zur Herstellung eines Wirkstoffabgabesystems, in dem, im ungehärteten Zustand und auf Gewichtsbasis, 2 bis 60% Glycerin, 2 bis 15% Polyvinylalkohol, 2 bis 10% eines wasserlöslichen Polymers mit Hydratisierungs-Zentren und als Rest Wasser mineinander vermischt werden, das erhaltene Gemisch erhitzt wird und die Mischung zur Bildung einer polymeren Diffusionsmatrix vergossen wird, und in dem eine therapeutisch wirksame Menge wenigstens eines für eine transdermale Verabreichung an einen Patienten geeigneten Medikaments der Mischung zugesetzt und in dieser dispergiert wird oder als Reservoir, das an der Oberfläche der Diffusionsmatrix angebracht ist, deponiert wird.

9. Verfahren nach Anspruch 8, das die folgenden Schritte umfaßt:

(a) Vermischen des Glycerins mit Wasser;
(b) Lösen des Polyvinylalkohols und des wasserlöslichen Polymers mit den Hydratisierungs-Zentren in der Mischung (a) durch Rühren und Erhitzen auf 90°C bis 95°C; und
(c) Vergießen der Mischung zur Bildung von Folien der Diffusionsmatrix,

wobei das Medikament der aus Schritt (b) resultierenden Mischung zugesetzt und in dieser dispergiert wird oder an der Oberfläche der Matrix abgelagert wird.

10. Verfahren nach Anspruch 9, das den Zusätzlichen Schritt des Härtens der in Schritt (c) erhaltenen Matrix einschließt, wodurch eine gehärtete Matrix erzeugt wird, die 2 bis 55% Glycerin, 4 bis 30% Polyvinylalkohol, 2 bis 20% des wasserlöslichen Polymers mit Hydratisierungs-Zentren und als Rest Wasser enthält.

11. Wirkstoffabgabesystem nach einem der Ansprüche 1 bis 6 oder hergestellt mittels eines Verfahrens nach einem der Ansprüche 8 bis 10 oder Wirkstoffabgabevorrichtung nach Anspruch 7 zur Verwendung bei der transdermalen Verabreichung wenigstens eines Medikaments an einen Patienten.

**Patentansprüche für den Vertragsstaat: AT**

1. Verfahren zur Herstellung eines Wirkstoffabgabesystems, in dem, im ungehärteten Zustand und auf Gewichtsbasis, 2 bis 60% Glycerin, 2 bis 15% Polyvinylalkohol, 2 bis 10% eines wasserlöslichen Polymers mit Hydratisierungs-Zentren und als Rest Wasser miteinander vermischt werden, das erhaltene Gemischerhitzt wird und die Mischung zur Bildung einer polymeren Diffusionsmatrix vergossen wird, und in dem eine therapeutisch wirksame Menge wenigstens eines für eine transdermale Verabreichung an einen Patienten geeigneten Medikaments der Mischung zugesetzt und in dieser dispergiert wird oder als Reservoir, das an der Oberfläche der Diffusionsmatrix angebracht ist, deponiert wird.

2. Verfahren nach Anspruch 1, das die folgenden Schritte umfaßt:

(a) Vermischen des Glycerins mit Wasser;
(b) Lösen des Polyvinylalkohols und des wasserlöslichen Polymers mit den Hydratisierungs-Zentren in der Mischung (a) durch Rühren und Erhitzen auf 90°C bis 95°C; und
(c) Vergießen der Mischung zur Bildung von Folien der Diffusionsmatrix,

wobei das Medikament der aus Schritt (b) resultierenden Mischung zugesetzt und in dieser dispergiert wird oder an der Oberfläche der Matrix abgelagert wird.

3. Verfahren nach Anspruch 1 oder Anspruch 2, dadurch gekennzeichnet, daß das wasserlösliche Polymer Agar, Agarose, Polyvinylpyrrolidon oder ein wasserlösliches Cellulose-Derivat ist.

4. Verfahren nach Anspruch 3, das den zusätzlichen Schritt des Härtens der in Schritt (c) erhaltenen Matrix einschließt, wodurch eine gehärtete Matrix erzeugt wird, die 2 bis 55% Glycerin, 4 bis 30% Polyvinylalkohol, 2 bis 20% des wasserlöslichen Polymers mit Hydratisierungs-Zentren und als Rest Wasser enthält.

5. Verfahren nach einem der Ansprüche 1 bis 4, dadurch gekennzeichnet, daß das Medikament Trinitroglycerin ist.

**O 013 606**

6. Verfahren nach Anspruch 5, dadurch gekennzeichnet, daß das Trinitroglycerin in der Mischung (b) dispergiert wird, wenn die Temperatur der Mischung auf 50°C bis 55°C gesenkt worden ist.

**Revendications pour les Etats contractants: BE, CH, DE, FR, GB, IT, LU, NL, SE**

1. Système de délivrance de médicament pour la libération prolongée d'un médicament, ledit système comprenant:

(A) une matrice de diffusion polymérique comprenant de 2 à 60% de glycérol, de 2 à 15% d'alcool polyvinylique, de 2 à 10% d'un polymère hydrosoluble à sites d'hydratation, le complément étant de l'eau, les pourcentages étant en poids, et
(B) une quantité thérapeutiquement efficace d'au moins un médicament applicable transdermiquement à un patient, ledit constituant médicamenteux (B) étant dispersé dans ladite matrice (A) ou étant déposé dans un réservoir de médicament apposé à ladite matrice (A).

2. Système de délivrance de médicament selon la revendication 1, dans lequel ledit médicament est du trinitroglycérol.

3. Système de délivrance de médicament selon la revendication 1, dans lequel ledit médicament est un antibiotique, un anesthésique local, un analgésique, un fongicide, un bactéricide ou un antimycoplasme.

4. Système de délivrance de médicament selon l'une quelconque des revendications 1 à 3, dans lequel la matrice est séchée et comprend de 2 à 55% de glycérol, d'environ 4 à 30% d'alcool polyvinylique, de 2 à 20% du polymère hydrosoluble à sites d'hydratation, et le complément étant de l'eau.

5. Système de délivrance de médicament selon l'une quelconque des revendications 1 à 4, dans lequel le polymère soluble dans l'eau est de la polyvinylpyrrolidone, de l'agar, de l'agarose ou un dérivé hydrosoluble de la cellulose.

6. Système de délivrance de médicament selon l'une quelconque des revendications 1 à 5, dans lequel l'alcool polyvinylique présente un poids moléculaire de 100.000 à 150.000, et la polyvinylpyrrolidone, lorsqu'elle est présente pour constituer le polymère hydrosoluble, a un poids moléculaire de 20.000 à 60.000.

7. Dispositif de délivrance de médicament comprenant un système de délivrance de médicament constitué par une matrice de diffusion polymérique (14) en association avec une quantité thérapeutiquement efficace d'au moins un médicament selon l'une quelconque des revendications 1 à 6, et un moyen (10) permettant de fixer ladite matrice pourvue dudit médicament à la peau d'un patient.

8. Procédé de confection d'un système de délivrance de médicament, qui comprend le mélange, dans un état non séché, en proportions pondérales, de 2 à 60% de glycérol, de 2 à 15% d'alcool polyvinylique, de 2 à 10% d'un polymère hydrosoluble à sites d'hydratation, le complément étant de l'eau, le chauffage du mélange résultant et le coulage du mélange de façon à former une matrice de diffusion polymérique, dans lequel une quantité thérapeutiquement efficace d'au moins un médicament applicable transdermiquement à un patient est ajoutée et dispersée dans le mélange ou déposée sous forme de réservoir fixé à la surface de ladite matrice de diffusion.

9. Procédé selon la revendication 8, comprenant les opérations suivantes:

(a) mélange du glycérol avec de l'eau;
(b) dissolution de l'alcool polyvinylique et du polymère hydrosoluble à sites d'hydratation dans le mélange de l'opération (a) par agitation et chauffage à 90 à 95°C; et
(c) coulage du mélange de façon à former des nappes de la matrice de diffusion,

ledit médicament étant ajouté à et dispersé dans le mélange résultant de l'opération (b), ou déposé à la surface de ladite matrice.

10. Procédé selon la revendication 9, comportant l'opération additionnelle de séchage de la matrice obtenue à l'opération (c) pour obtenir une matrice séchée comprenant, en proportions pondérales, de 2 à 55% de glycérol, de 4 à 30% d'alcool polyvinylique, de 2 à 20% du polymère hydrosoluble à sites d'hydratation, le complément étant de l'eau.

11. Système de délivrance de médicament selon l'une quelconque des revendications 1 à 6, ou obtenu par un procédé selon l'une quelconque des revendications 8 à 10, ou dispositif de délivrance de médicament selon la revendication 7, pour utilisation dans l'administration transdermique à un patient d'au moins un médicament.

**Revendications pour l'Etat contractant: AT**

1. Procédé de confection d'un système de délivrance de médicament, comprenant le mélange, dans un état non séché et en proportions pondérales, de 2 à 60% de glycérol, de 2 à 15% d'alcool

12

**0 013 606**

polyvinylique, de 2 à 10% d'un polymère hydrosoluble à sites d'hydratation, le complément étant de l'eau, le chauffage du mélange résultant et le coulage du mélange de façon à former une matrice polymérique de diffusion, dans laquelle une quantité thérapeutiquement efficace d'au moins un médicament applicable transdermiquement à un patient est ajoutée au mélange et dispersée dans celui-ci ou déposée sous forme de réservoir fixé à la surface de ladite matrice de diffusion.

2. Procédé selon la revendication 1, comprenant les opérations suivantes:

(a) mélange du glycérol avec de l'eau;
(b) dissolution de l'alcool polyvinylique et du polymère hydrosoluble à sites d'hydratation dans le mélange de l'opération (a) par agitation et chauffage à 90 à 95°C; et
(c) coulage du mélange de façon à former des nappes de la matrice de diffusion,

ledit médicament étant ajouté à et dispersé dans le mélange résultant de l'opération (b), ou déposé à la surface de ladite matrice.

3. Procédé selon la revendication 1 ou 2, dans lequel le polymère hydrosoluble est de l'agar, de l'agarose, de la polyvinylpyrrolidone ou un dérivé hydrosoluble de la cellulose.

4. Procédé selon la revendication 3, comportant l'opération additionnelle de séchage de la matrice obtenue à l'opération (c) pour obtenir une matrice séchée comprenant, en proportions pondérales, de 2 à 55% de glycérol, de 4 à 30% d'alcool polyvinylique, de 2 à 20% du polymère hydrosoluble à sites d'hydratation, et le complément étant de l'eau.

5. Procédé selon l'une quelconque des revendications 1 à 4, dans lequel le médicament est du trinitroglycérol.

6. Procédé selon la revendication 5, dans lequel ledit trinitroglycérol est dispersé dans le mélange de l'opération (6) lorsque la température du mélange a été abaissée à 50 à 55°C.

13

Fig.1

Fig.2